# EUROPEAN PATENT SPECIFICATION

(11) **EP 2 451 465 B1**
(45) Date of publication and mention of the grant of the patent: **23.08.2017**
(21) Application number: 10797690.4
(22) Date of filing: 02.07.2010
(51) Int. Cl.: A61K 35/16, G01N 33/68

(54) **PHENOTYPIC RATIO OF SERUM AMYLOID IN PRE- AND TYPE 2 DIABETES**
PHÄNOTYPISCHES SERUMAMYLOIDVERHÄLTNIS BEI PRÄDIABETES UND DIABETES VOM TYP 2
RAPPORT PHÉNOTYPIQUE D'AMYLOÏDE SÉRIQUE DANS LE PRÉDIABÈTE ET LE DIABÈTE DE TYPE 2

(30) Priority: 07.07.2009 US 223498 P
(43) Date of publication of application: 16.05.2012
(73) Proprietor: Intrinsic Bioprobes, Inc., Tempe, AZ 85284 (US)
(72) Inventor: KIERNAN, Urban, A., Chandler AZ 85249 (US); NEDELKOV, Dobrin, Tempe AZ 85284 (US)
(74) Representative: Hanna Moore + Curley
(86) International application number: PCT/US2010/040997
(87) International publication number: WO 2011/005717

(56) References cited:
- WO-A1-2009/006568
- US-A1- 2007 087 448
- US-A1- 2008 039 364
- US-A1- 2009 012 716
- KUMON Y ET AL: "Serum amyloid A protein in patients with non-insulin-dependent diabetes mellitus", CLINICAL BIOCHEMISTRY, ELSEVIER INC, US, CA, vol. 27, no. 6, 1 December 1994 (1994-12-01), pages 469-473, XP025201487, ISSN: 0009-9120, DOI: 10.1016/0009-9120(94)00044-V [retrieved on 1994-12-01]
- HATANAKA ET AL.: 'Interaction between serum amyloid A and leukocytes - A possible role in the progression of vascular complications in diabetes.' IMMUNOLOGY LETTERS vol. 108, 2007, pages 160 - 166, XP005876102
- KIERNAN ET AL.: 'Detection of novel truncated forms of human serum amyloid A protein in human plasma.' FEBS LETTERS vol. 537, 2003, pages 166 - 170, XP004411998

## Description

### FIELD OF THE INVENTION

This invention relates to methods of determining, diagnosing, and/or assessing pre-diabetes, type 2 diabetes, insulin resistance and their related conditions by detecting levels and modulations of the phenotypic ratio of Serum amyloid A protein (SAA). This invention also relates to methods for screening molecules that modulate this ratio and their use in the treatment and/or amelioration of symptoms that are related to pre-diabetes, type 2 diabetes, insulin resistance and their related conditions.

### BACKGROUND OF THE INVENTION

Type 2 diabetes (also known as diabetes mellitus type 2, non-insulin-dependent diabetes mellitus or adult on-set diabetes) is a metabolic disorder that is characterized by high blood glucose. This condition is a result of either a lack of insulin produced by the body or the developed tolerance to insulin by the cells of the body (Insulin Resistance). Since insulin is essential for the absorption of glucose from the blood, perturbations in insulin homeostasis eventually result in loss of glycemic control and the development of the diabetic condition. If left uncontrolled, excessive blood glucose will eventually lead to a multitude of complications, including but not limited to: blindness, skin ulcerations, amputations, heart disease and kidney disease. Many proposed theories exist regarding the association of multiple complications that are linked to the loss of glycemic control, however, the mechanisms in which these multiple pathways interact is still unknown.

As of 2007, the Centers of Disease Control determined that 7.8% of the US population (23.6 million people) have type 2 diabetes and it is estimated that >20% of Americans are pre-diabetic. Due to the debilitating nature of these conditions and their associated cost of treatment, more effective and efficient methods for earlier detection are paramount to the health of the US populace and western civilization. The "gold standard" for evaluating the level of glycemic control is the Oral Glucose Tolerance Test (OGTT). This test involves measuring the fasting blood glucose of an individual or patient and then obtaining a blood glucose measurement 2 hours post administration of an oral glucose solution. Current guidelines define normal glucose tolerance (NGT) as a 2 hour glucose level ≤ 140 mg/dl. The criteria for type 2 diabetes (OM) is a 2 hour glucose of ≥ 200 mg/dl and the definition of impaired glucose tolerance (IGT or pre-diabetes) is between 140 and 200 mg/dl. Even though these criteria are sanctioned by both the American Diabetes Association and the World Health Organization, these values are not without clinical error or subject to interpretation.

A recent development in diabetes care included the adoption of guidelines for the use of the protein biomarker hemoglobin A1C (HbA1C) in the diagnosis of diabetes and pre-diabetes. Due to the longevity of hemoglobin in the blood, it serves as a long term measure of glycemic control. Although long used as a biomarker of whether or not a patient's diabetes treatment is sufficient, conflicting data previously prevented the use of HbA1C in diabetes determination. Even with the acceptance of these new guidelines, and their use in conjunction with the standard OGTT criteria and fasting blood glucose levels, the diagnosis of pre- or type 2 diabetes still ultimately relies on the interpretation and the judgment of the diagnosing physician.

Due to the pandemic nature of type 2 diabetes, pre-diabetes, insulin resistance and the development of associated complications, there is a world wide need for additional detection/diagnostic/assessment methods to allow for the earliest possible intervention and provide a means to evaluate the effectiveness of treatment through life-style changes and/or medication. A need also exists for additional metabolic or endocrine targets for the development of treatments that alleviate or ameliorate the problems and symptoms associated with these related conditions.

### SUMMARY OF THE INVENTION

The present teaching discloses a method for diagnosing at least one of impaired glucose tolerance and type 2 diabetes in a biological sample of a subject as detailed in claim 1.

As shown here for the first time, modulations in the phenotypic ratio of SAA correlate with the 2 hour glucose values obtained after the application of an OGTT and the clinical diagnosis of certain metabolic disease states. These findings identify the phenotypic ratio of SAA as a biomarker for the clinical assessment/diagnosis of type 2 diabetes, pre- diabetes, insulin resistance and
their related conditions. It also stands to reason that SAA may also mechanistically participate in the initiation/development and pathology of these disease states and therefore the phenotypic ratio of SAA may also have utility as a therapeutic target for the amelioration and/or treatment of the disease symptoms or condition.

Another objective of the present invention is to use SAA and the calculation of the phenotypic ratio of SAA as new targets and screening methods for the in-vitro and in-vivo detection and/or diagnosis of type 2 diabetes, pre-diabetes, insulin resistance and their related conditions.

A further objective of the present invention is to use the phenotypic ratio of SAA as a new target for molecules that modulate SAA and/or in the screening of molecules aimed at the in-vitro and/or in-vivo modulation of its expression, activity and/or clearance in the treatment of Type 2 diabetes, pre-diabetes, insulin resistance and their related conditions.

Some novel features that are considered characteristic of the invention are set forth with particularity in the claims. The invention itself, however, both as to its structure and its operation together with the additional objectives and advantages thereof will best be understood from the following description of the exemplary embodiments of the present invention when read in conjunction with the accompanying drawings. Unless specifically noted, it is intended that the words and phrases in the specification and claims be given the ordinary and accustomed meaning for those of ordinary skill in the application of the art or arts. If any other meaning is intended, the specification will specifically state that special meaning is being applied to the word or phrase. Likewise, the use of the words "function" or "means" in the Description of Preferred Embodiments is not intended to indicate a desire to invoke the special provisions of 35 U.S.C. § 112, paragraph 6 to define the invention. To the contrary, if the provisions of 35 U.S.C. § 112, paragraph 6, are sought to be invoked to define the invention(s), the claims will specifically state the phrases "means for" or "step for" and a function, without also reciting in such phrases any structure, material, or act in support of the function. Even when the claims recite a "means for" or "step for" performing a function, if they also recite any structure, material or acts in support of that means or step, then the intention is not to invoke the provisions of 35 U.S.C. § 112, paragraph 6. Moreover, even if the provisions of 35 U.S.C. § 112, paragraph 6, are invoked to define the inventions, it is intended that the inventions not be limited only to the specific structure, material or acts that are described in the preferred embodiments, but in addition, include all structures, materials or acts that perform the claimed function, along with any known or later-developed equivalent structures, materials or acts for performing the claimed function.

### BRIEF DESCRIPTION OF THE DRAWINGS

The object and features of the present invention will be better understood with reference to the following detailed description and drawings.
FIG. 1 illustrates mass spectral results of the analysis of SAA and the phenotypic ratio of SAA from human plasma samples using mass spectrometric immunoassay.
FIG. 2 is a dot histogram of the observed phenotypic ratio of SAA from the human plasma samples from patients with normal glucose tolerance (NGT) and patients with impaired glucose tolerance (IGT, pre-diabetes) that were analyzed and shown in FIG. 1.
FIG. 3 is a dot histogram of the observed phenotypic ratio of SAA from the human plasma samples from patients with normal glucose tolerance (NGT) and patients with type 2 diabetes (DM) that were analyzed and shown in FIG. 1.
FIG. 4 shows the resultant ROC curves established from the mass spectral phenotypic ratio of SAA data obtained from each sample population, namely the human plasma samples from patients with normal glucose tolerance (NGT), the human plasma samples from patients with impaired glucose tolerance (IGT, pre-diabetes), and the human plasma samples from patients with type 2 diabetes (DM), that were analyzed and shown in FIG. 1.

### DETAILED DESCRIPTION

It is demonstrated for the first time in the present invention that the phenotypic ratio of SAA correlates with the clinical diagnosis of both the pre-diabetes and type 2 diabetes disease states. It is also demonstrated for the first time that the modulations in the phenotypic ratio of SAA compare to the results of the OGTT. This observed phenomenon directly displays the utility of this novel marker as it relates to type 2 diabetes, pre-diabetes, insulin resistance and/or their related conditions or complications.

The present invention encompasses the use of the phenotypic ratio of SAA as a biomarker for the in-vitro and/or in-vivo determination/diagnosis/assessment of type 2 diabetes, pre-diabetes, insulin resistance and/or their related conditions or complications.

In addition, the present invention includes the use of the phenotypic ratio of SAA as a marker for the in-vitro and/or in-vivo screening of compounds, aimed at modulating this ratio by altering the expression, modification, activity and/or clearance of SAA for the purpose of treating, or reducing symptoms that are associated with, pre-diabetes, type 2 diabetes, insulin resistance and/or their related conditions or complications.

The present invention also includes methodologies for the measurement of the forms of SAA for the calculation of the phenotypic ratio of SAA from biological samples for the determination/diagnosis/assessment of type 2 diabetes, pre-diabetes, insulin resistance and/or their related conditions or complications.

The present invention also extends these methodologies for the determination of the phenotypic ratio of SAA in order to screen compounds aimed at modulating this ratio by altering the expression, activity and/or clearance of SAA for the purpose of treating, or reducing symptoms that are associated with, pre-diabetes, type 2 diabetes, insulin resistance and/or their related conditions or complications.

### Definitions

All the technical and scientific terms used herein, unless defined otherwise, have the meaning commonly understood by a person skilled in art to which this invention pertains. As used herein, the following terms have the meaning ascribed to them unless specified otherwise.

As used herein, "SAA" is a general term describing the serum amyloid A acute phase family of proteins. There are two isoforms of SAA, which are expressed by the genes SAA1 and SAA2, with multiple allelic variations of each. These are collectively known as SAA. SAA serves as an apolipoprotein and is expressed in both hepatocytes and adipocytes.

As used herein, "Parent SAA" refers to the most common intact endogenous form of SAA observed within the sample. This is normally SAA1 (MW = 11,683) but may also include an allelic variant or a point mutated forms.

As used herein, "SAA-Arg" refers to the specific truncated variant of SAA which is missing its N-terminal Arginine residue.

As used herein, "Analysis" refers to the determination of the phenotypic ratio of SAA from a biological sample.

As used herein, "Phenotypic ratio of SAA" refers to the quotient calculated by dividing the measured amount of parent SAA by the measured amount of SAA-Arg from a given biological sample.

As used herein, "Biological sample" refers to a fluid or extract having a plurality of components. Complex media may include, but is not limited to, tissue, cell extracts, nuclear extracts, cell lysates and excretions, blood, sera, plasma, saliva, urine, sputum, synovial fluid, cerebral-spinal fluid, tears, feces, saliva, membrane extracts, industrial fluids and the like.

As used herein, "type 2 diabetes (DM)" is a clinical condition defined by excess glucose in the blood. Reference limits that aid in the clinical determination are a Fasting glucose value ≥126 mg/dl and/or a 2 hour oral glucose tolerance test value ≥ 200 mg/dl.

As used herein, "pre-diabetes, impaired glucose tolerance (IGT)" is a clinical condition defined by above normal amounts of glucose present in the blood. Reference limits that aid in the clinical determination are Fasting glucose values that are between 110 and 125 mg/dl and/or 2 hour oral glucose tolerance test values that are between 140 and 199 mg/dl.

As used herein, "normal glucose tolerance (NGT)" is a clinical determination when blood glucose levels are within normal concentration ranges. Reference limits that aid in the clinical determination are a Fasting glucose value < 110 mg/dl and/or a 2 hour oral glucose tolerance test value < 140 mg/dl.

As used herein, "insulin resistance" is a physical condition in which the hormone insulin becomes less effective in reducing blood glucose levels. The gold standard for evaluating insulin resistance is the administration of a hyperinsulinemic euglycemic clamp.

As used herein, "related conditions or complications" is defined as a variety of ailments that are commonly associated with the development and progression of type 2 diabetes. Some of these ailments include but are not limited to: heart attacks, skin ulcerations, blindness, amputations and kidney failure.

As used herein, "mass spectrometry" refers to the ability to volatilize/ionize analytes to form vapor-phase ions and determine their absolute or relative molecular masses. Suitable forms of volatilization/ionization are laser/light, thermal, electrical, atomized/sprayed and the like or combinations thereof. Suitable forms of mass spectrometry include, but are not limited to, Matrix Assisted Laser Desorption/Time of Flight Mass Spectrometry (MALDI-TOF MS), electrospray (or nanospray) ionization (ESI) mass spectrometry, or the like or combinations thereof.

As used herein, "subject" refers to any human, animal, or other living organism which possesses measurable levels of SAA and/or SAA variants in a biological sample taken therefrom.

In the present invention, the method for the establishment of the phenotypic ratio of SAA is performed using a biological sample (biological matrix). Possible biological matrices include, but are not limited to; tissue, whole blood, serum, plasma, saliva, cerebral spinal fluid or urine. The sample may also be free or immobilized onto a solid support. Examples of solid supports include, but are not limited to, filter paper, beads, arrays, etc. Any solid support material known in the art for immobilizing samples may be used. The sample may also be native in form or have undergone processing including, but not limited to, denaturation, reduction, proteolytic digestion and the like.

In order to measure the phenotypic ratio of SAA, the present invention may utilize a separation or purification step to first isolate or remove SAA from the biological sample. This separation can be performed by several means, including, but not limited to, chromatography, centrifugation, affinity interactions, chemical methodologies, staining methodologies, and gel electrophoresis. Chromatography techniques may include but are not limited to: high performance liquid chromatography (HPLC) thin layer chromatography (TLC), paper chromatography (PC), affinity chromatography, size exclusion, ion-exchange, reverse phase, etc. Affinity interactions employ the ability of a molecule to bind with another molecule having proper fitting conformation. Affinity methods employed may include, but are not limited to, affinity interactions utilizing antibodies (Ab), antibody fragments (FAb), aptamers, proteins, peptides, lectins, etc. For sandwich assays, combinations of primary and secondary affinity ligands may be used. Gel electrophoresis methods may include, but are not limited to, acrylamide, agarose, etc. Chemical methodologies may include, but are not limited to, amino acid analysis, sequencing, etc. Staining methods may include, but are not limited to, the use of any dye, and the like, that directly binds to the SAA protein and its variants, or to molecules that bind to SAA and its variants.

The method for detecting the forms of SAA used to calculate the phenotypic ratio of SAA will utilize some form of detection technology for the isolated protein (i.e. isolated SAA and/or SAA variants). Detection methods may be direct or indirect in nature. Detection technologies include but are not limited to: staining, spectroscopy, spectrometroscopy, spectrophotometry, colorimetry, fluorescence, luminescence, magnetism, radioisotopes, nuclear magnetic resonance spectroscopy, x-ray crystallography, surface plasmon resonance, mass spectrometry, etc. Depending on the state of the biological sample, the detected forms of SAA measured and used to calculate the phenotypic ratio of SAA may be intact or a fragment thereof.

### Analyses Demonstrating Biomarker Utility

### EXAMPLE

### ANALYSIS OF THE PHENOTYPIC RATIO OF SERUM AMYLOID A FROM HUMAN PLASMA

One exemplary embodiment of the invention is the measurement of the phenotypic ratio of SAA for use as a biomarker(s) to differentiate between clinically defined populations of samples. The analysis of this exemplary embodiment was performed using mass spectrometric immunoassay (MSIA) technology.

In this example, the analyses were performed on human citrate plasma samples obtained from patients that were newly diagnosed and clinically defined as having NGT, IGT or DM. There were 237 plasma samples obtained from NGT individuals, 98 plasma samples obtained from patients with IGT, and 25 plasma samples from patients with DM which were all analyzed in the same manner.

The MSIA was performed with the aid of affinity pipette tips. The affinity pipette tips are made of small, porous microcolumns that are fitted at the entrance of a pipettor tip. The microcolumns are derivatized with an affinity ligand. The affinity ligand used to derivatize the affinity pipettes was anti-SAA antibody. The sample preparation utilized the addition of an internal reference standard (IRS) for the semi-quantitative measurement of the parent SAA and SAA-Arg. Each sample mixture consisted of 10 µL of human plasma, 20 µL of ten-fold diluted horse serum (horse SAA is the IRS) and 170 µL of HEPES buffered saline. SAA was extracted from the samples by interrogation with the anti-SAA affinity pipette tips by repetitive drawing of the samples through the affinity pipettes. The purification process used HEPES buffered saline and water rinses following the SAA affinity retrieval. Enriched and purified proteins were then eluted from the affinity pipettes with sinapic acid MALDI matrix and were deposited directly on a MALDI mass spectrometer target for subsequent mass spectral detection.

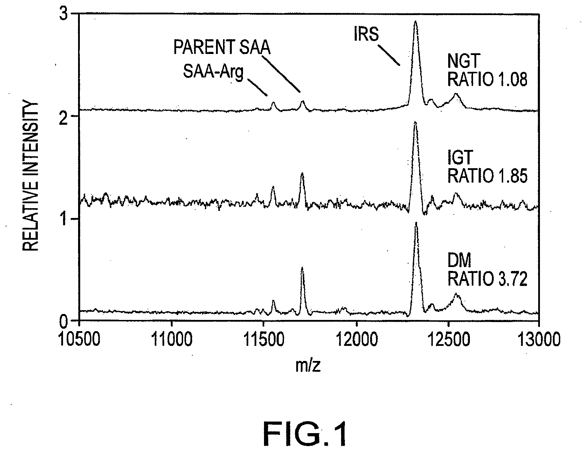

Examination of the resultant mass spectra showed signals from multiple forms of SAA that are present in human plasma, as well as the IRS. Each spectrum was normalized to the integral of the SAA signal obtained from the IRS. Within each mass spectrum, multiple forms of SAA are clearly resolved, but are dominated by the parent SAA and SAA-Arg as shown in FIG. 1. FIG. 1 also illustrates that the phenotypic ratio of SAA is different in human plasma samples from patients with normal glucose tolerance (NGT), impaired glucose tolerance (IGT) and type 2 diabetes (DM). The integral of the parent SAA and SAA-Arg signals were collected and the phenotypic ratio of SAA was calculated. The phenotypic ratio of SAA was established by taking the integral for the parent SAA and dividing it by the integral of the SAA-Arg. The ratio of SAA-Arg to parent SAA is clearly skewed in the IGT and DM spectra.

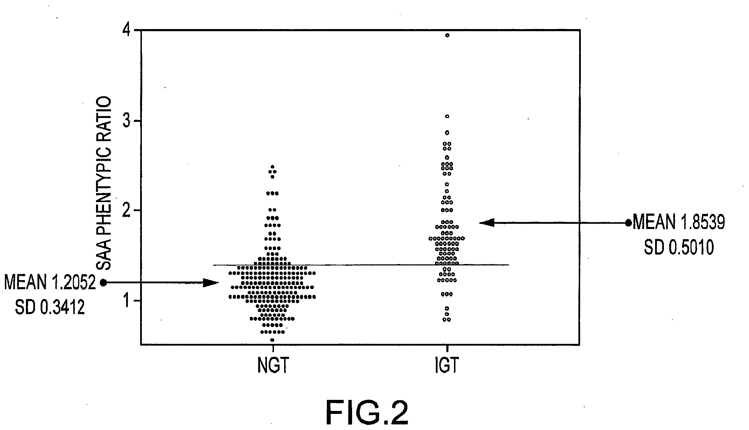

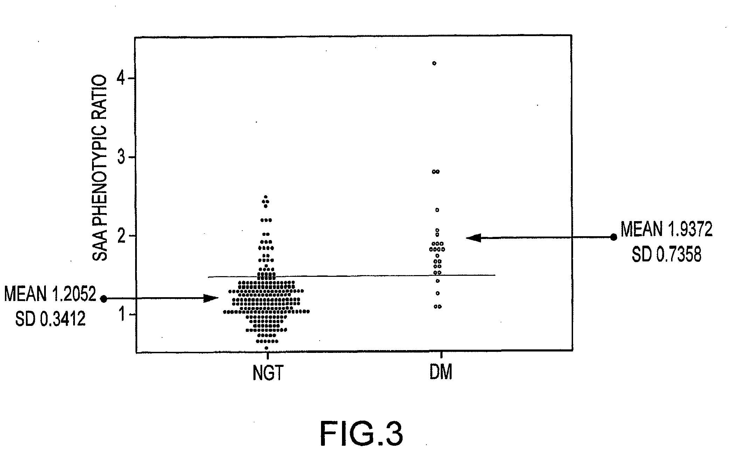

The resulting mass spectrometry data showed the ability to discriminate between samples that originate from NGT patients and IGT/DM patients. FIG. 2 is a dot histogram of the observed phenotypic ratio of SAA from the samples of patients having NGT and IGT. Using the phenotypic ratio of SAA as a biomarker for IGT, a cut-off value of 1.3760 was established for IGT determination. The mean value and one standard deviation of each population are also denoted in FIG. 2. FIG. 3 is a dot histogram of the observed phenotypic ratio of SAA from the samples of patients having NGT and DM. Using the phenotypic ratio of SAA as a biomarker for DM, a cut-off value of 1.4780 was established for DM determination. The mean value and one standard deviation of each population are also denoted in FIG. 3.

The clinical sensitivity and specificity for the phenotypic ratio of SAA were also determined. These values are presented below in **Table 1.**

| **Biomarker** | **Sensitivity** | **Specificity** | **Cut-off Value** |
|---|---|---|---|
| **IGT** | | | |
| SAA ratio | 80.4% | 80.3% | 1.3760 |

| **DM** | | | |
|---|---|---|---|
| SAA ratio | 84.0% | 87.0% | 1.4780 |

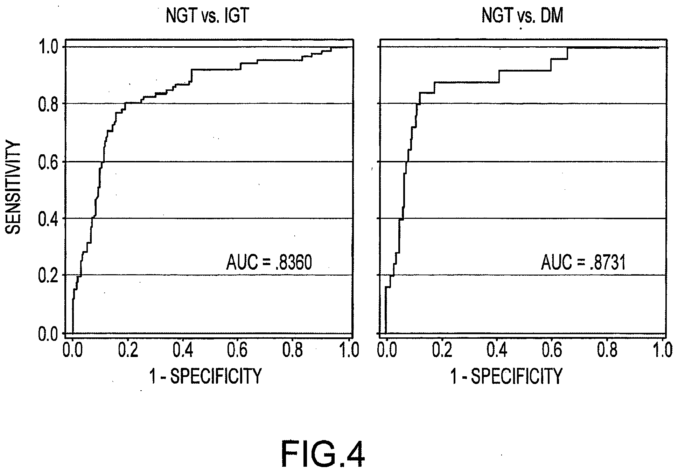

The accuracy of a test to discriminate diseased cases from normal cases is elevated using Receiver Operating Characteristic (ROC) curve analysis. For comparative purposes the receiver operator characteristic (ROC) curves of phenotypic ratio of SAA for IGT and DM were also plotted. These plots are shown in **FIG. 4****.** The displayed plots illustrate the clinical utility of the SAA phenotypic ratio biomarker in determining/diagnosing both IGT and DM. The calculated areas under the curve for each plot are 0.8360 and 0.8731, respectively.

Finally, an algorithm-assisted bio-statistical evaluation of the SAA phenotypic ratio dataset was performed, resulting in improved clinical sensitivity and specificity for determining/diagnosing both IGT and DM. This data is presented below in **Table 2.**

| **Biomarker** | **Sensitivity** | **Specificity** |
|---|---|---|
| **IGT** | | |
| SAA ratio | 87.0% | 79.0% |

| **DM** | | |
|---|---|---|
| SAA ratio | 100% | 91.1% |

All of the analyses set forth and/or described above can also be performed on other SAA variants and/or applied to combinations of various observed SAA variants to determine additional biomarkers or groupings of biomarkers for determining IGT, DM and their related conditions.

The preferred embodiment of the invention is described above in the Drawings and Description of Preferred Embodiments. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventors that the words and phrases in the specification and claims be given the ordinary and accustomed meanings to those of ordinary skill in the applicable art(s). The foregoing description of an exemplary embodiment and best mode of the invention known to the applicant at the time of filing the application has been presented and is intended for the purposes of illustration, and description. It is not intended to be exhaustive or to limit the invention to the precise form disclosed, and many modifications and variations are possible in the light of the above teachings. The embodiment was chosen and described in order to best explain the principles of the invention and its practical application and to enable others skilled in the art to best utilize the invention in various embodiments and with various modifications as are suited to the particular use contemplated.

## Claims

1. A method for diagnosing at least one of impaired glucose tolerance and type 2 diabetes in a biological sample of a subject comprising the steps of determining a phenotypic ratio of SAA in the biological sample of the subject by dividing a detected amount of parent SAA present in the biological sample by a detected amount of SAA-Arg present in the biological sample and diagnosing said subject as having at least one of impaired glucose tolerance and type 2 diabetes when the phenotypic ratio of SAA in the biological sample of the subject is modulated by a statistically significant amount from a phenotypic ratio of SAA present in a biological sample of one or more subjects with normal glucose tolerance.

2. The method of claim 1 wherein the step of determining a phenotypic ratio of SAA in the biological sample of the subject comprises the step of performing at least one of mass spectroscopy, chromatography, an affinity reaction, gel electrophoresis, centrifugation, chemical methodology, and staining methodology.

3. The method of claim 1 wherein the step of determining a phenotypic ratio of SAA in the biological sample of the subject comprises the step of performing a single assay.

4. The method of claim 3 wherein the step of performing a single assay comprises the step of performing single mass spectrometry.

5. The method of claim 1 wherein the step of diagnosing said subject as having at least one of impaired glucose tolerance and type 2 diabetes when the phenotypic ratio of SAA is modulated by a statistically significant amount over a phenotypic ratio of SAA present in one or more subjects with normal glucose tolerance comprises the step of diagnosing the subject as having impaired glucose tolerance when the phenotypic ratio of the SAA in the biological sample of the subject is at least 1.3760.

6. The method of claim 1 wherein the step of diagnosing said subject as having at least one of impaired glucose tolerance and type 2 diabetes when the phenotypic ratio of SAA is modulated by a statistically significant amount over a phenotypic ratio of SAA present in one or more subjects with normal glucose tolerance comprises the step of diagnosing the subject as having type 2 diabetes when the phenotypic ratio of the SAA in the biological sample of the subject is at least 1.4780.

7. The method of claim 1 wherein the step of diagnosing said subject as having at least one of impaired glucose tolerance and type 2 diabetes comprises the step of diagnosing said subject as having at least one of impaired glucose tolerance and type 2 diabetes when the phenotypic ratio of SAA in the biological sample of the subject is increased by a statistically significant amount over a level present in the biological sample of one or more subjects with normal glucose tolerance.

8. The method of claim 7 wherein the step of diagnosing said subject as having at least one of impaired glucose tolerance and type 2 diabetes when the phenotypic ratio of SAA is modulated by a statistically significant amount over a phenotypic ratio of SAA present in one or more subjects with normal glucose tolerance comprises the step of diagnosing the subject as having impaired glucose tolerance when the phenotypic ratio of the SAA in the biological sample of the subject is at least 1.3760.

9. The method of claim 7 wherein the step of diagnosing said subject as having at least one of impaired glucose tolerance and type 2 diabetes when the phenotypic ratio of SAA is modulated by a statistically significant amount over a phenotypic ratio of SAA present in one or more subjects with normal glucose tolerance comprises the step of diagnosing the subject as having type 2 diabetes when the phenotypic ratio of the SAA in the biological sample of the subject is at least 1.4780.

10. The method of claim 8 wherein the step of determining a phenotypic ratio of SAA in the biological sample of the subject comprises the step of performing a single assay.

11. The method of claim 10 wherein the step of performing a single assay comprises the step of performing single mass spectrometry.

12. The method of claim 9 wherein the step of determining a phenotypic ratio of SAA in the biological sample of the subject comprises the step of performing a single assay.

13. The method of claim 12 wherein the step of performing a single assay comprises the step of performing single mass spectrometry.

## Patentansprüche

1. Verfahren zur Diagnose von mindestens einem von beeinträchtigter Glukosetoleranz und Diabetes vom Typ 2 in einer biologischen Probe eines Individuums, umfassend die Schritte des Ermittelns eines phänotypischen Verhältnisses von SAA in der biologischen Probe des Individuums durch das Dividieren einer detektierten Menge von Eltern-SAA, die in der biologischen Probe vorliegt, durch eine detektierte Menge von SAA-Arg, die in der biologischen Probe vorliegt, und des Diagnostizierens, dass das Individuum mindestens eines von einer beeinträchtigten Glukosetoleranz und Diabetes vom Typ 2 aufweist, wenn das phänotypische Verhältnis von SAA in der biologischen Probe des Individuums durch eine statistisch signifikante Menge von einem phänotypischen Verhältnis von SAA moduliert wird, das in einer biologischen Probe von einem oder mehreren Individuen mit einer normalen Glukosetoleranz vorliegt.

2. Verfahren nach Anspruch 1, wobei der Schritt des Ermittelns eines phänotypischen Verhältnisses von SAA in der biologischen Probe des Individuums den Schritt des Durchführens von mindestens einem von einer Massenspektroskopie, einer Chromatographie, einer Affinitätsreaktion, einer Gelelektrophorese, einer Zentrifugation, chemischen Methoden und Färbemethoden umfasst.

3. Verfahren nach Anspruch 1, wobei der Schritt des Ermittelns eines phänotypischen Verhältnisses von SAA in der biologischen Probe des Individuums den Schritt des Durchführens eines einzelnen Assays umfasst.

4. Verfahren nach Anspruch 3, wobei der Schritt des Durchführens eines einzelnen Assays den Schritt des Durchführens einer einzelnen Massenspektrometrie umfasst.

5. Verfahren nach Anspruch 1, wobei der Schritt des Diagnostizierens, dass das Individuum mindestens eines von einer beeinträchtigen Glukosetoleranz und Diabetes vom Typ 2 aufweist, wenn das phänotypische Verhältnis von SAA durch eine statistisch signifikante Menge über ein phänotypisches Verhältnis von SAA moduliert wird, das in einem oder mehreren Individuen mit einer normalen Glukosetoleranz vorliegt, den Schritt des Diagnostizierens umfasst, dass das Individuum eine beeinträchtigte Glukosetoleranz aufweist, wenn das phänotypische Verhältnis von dem SAA in der biologischen Probe des Individuums mindestens 1,3760 beträgt.

6. Verfahren nach Anspruch 1, wobei der Schritt des Diagnostizierens, dass das Individuum mindestens eines von einer beeinträchtigen Glukosetoleranz und Diabetes vom Typ 2 aufweist, wenn das phänotypische Verhältnis von SAA durch eine statistisch signifikante Menge über ein phänotypisches Verhältnis von SAA moduliert wird, das in einem oder mehreren Individuen mit einer normalen Glukosetoleranz vorliegt, den Schritt des Diagnostizierens umfasst, dass das Individuum Diabetes vom Typ 2 aufweist, wenn das phänotypische Verhältnis von dem SAA in der biologischen Probe des Individuums mindestens 1,4780 beträgt.

7. Verfahren nach Anspruch 1, wobei der Schritt des Diagnostizierens, dass das Individuum mindestens eines von einer beeinträchtigen Glukosetoleranz und Diabetes vom Typ 2 aufweist, den Schritt des Diagnostizierens umfasst, dass das Individuum mindestens eines von einer beeinträchtigten Glukosetoleranz und Diabetes vom Typ 2 aufweist, wenn das phänotypische Verhältnis von SAA in der biologischen Probe des Individuums um eine statistisch signifikante Menge über einem Gehalt erhöht ist, der in der biologischen Probe von einem oder mehreren Individuen mit einer normalen Glukosetoleranz vorliegt.

8. Verfahren nach Anspruch 7, wobei der Schritt des Diagnostizierens, dass das Individuum mindestens eines von einer beeinträchtigten Glukosetoleranz und Diabetes vom Typ 2 aufweist, wenn das phänotypische Verhältnis von SAA durch eine statistisch signifikante Menge über ein phänotypisches Verhältnis von SAA moduliert wird, das in einem oder mehreren Individuen mit einer normalen Glukosetoleranz vorliegt, den Schritt des Diagnostizierens umfasst, dass das Individuum eine beeinträchtigte Glukosetoleranz aufweist, wenn das phänotypische Verhältnis von dem SAA in der biologischen Probe des Individuums mindestens 1,3760 beträgt.

9. Verfahren nach Anspruch 7, wobei der Schritt des Diagnostizierens, dass das Individuum mindestens eines von einer beeinträchtigten Glukosetoleranz und Diabetes vom Typ 2 aufweist, wenn das phänotypische Verhältnis von SAA durch eine statistisch signifikante Menge über ein phänotypisches Verhältnis von SAA moduliert wird, das in einem oder mehreren Individuen mit einer normalen Glukosetoleranz vorliegt, den Schritt des Diagnostizierens umfasst, dass das Individuum Diabetes vom Typ 2 aufweist, wenn das phänotypische Verhältnis von dem SAA in der biologischen Probe des Individuums mindestens 1,4780 beträgt.

10. Verfahren nach Anspruch 8, wobei der Schritt des Ermittelns eines phänotypischen Verhältnisses von SAA in der biologischen Probe des Individuums den Schritt des Durchführens eines einzelnen Assays umfasst.

11. Verfahren nach Anspruch 10, wobei der Schritt des Durchführens eines einzelnen Assays den Schritt des Durchführens einer einzelnen Massenspektrometrie umfasst.

12. Verfahren nach Anspruch 9, wobei der Schritt des Ermittelns eines phänotypischen Verhältnisses von SAA in der biologischen Probe des Individuums den Schritt des Durchführens eines einzelnen Assays umfasst.

13. Verfahren nach Anspruch 12, wobei der Schritt des Durchführens eines einzelnen Assays den Schritt des Durchführens einer einzelnen Massenspektrometrie umfasst.

## Revendications

1. Procédé de diagnostic d'au moins un parmi une tolérance altérée au glucose et un diabète de type 2 dans un échantillon biologique d'un sujet comprenant les étapes de détermination d'un rapport phénotypique de SAA dans l'échantillon biologique du sujet par division d'une quantité détectée de SAA parent présent dans l'échantillon biologique par une quantité détectée de SAA-Arg présent dans l'échantillon biologique et de diagnostic du sujet comme ayant au moins l'un de la tolérance altérée au glucose et du diabète de type 2 lorsque le rapport phénotypique de SAA dans l'échantillon biologique du sujet est modulé par une quantité statistiquement significative par rapport à un rapport phénotypique de SAA présent dans un échantillon biologique d'un ou de plusieurs sujets ayant une tolérance au glucose normale.

2. Procédé selon la revendication 1, dans lequel l'étape de détermination d'un rapport phénotypique de SAA dans l'échantillon biologique du sujet comprend l'étape de réalisation d'au moins l'une d'une spectrométrie de masse, d'une chromatographie, d'une réaction d'affinité, d'une électrophorèse sur gel, d'une centrifugation, d'une méthodologie chimique et d'une méthodologie de coloration.

3. Procédé selon la revendication 1, dans lequel l'étape de détermination d'un rapport phénotypique de SAA dans l'échantillon biologique du sujet comprend l'étape de réalisation d'un seul dosage.

4. Procédé selon la revendication 3, dans lequel l'étape de réalisation d'un seul dosage comprend l'étape de réalisation d'une seule spectrométrie de masse.

5. Procédé selon la revendication 1, dans lequel l'étape de diagnostic dudit sujet comme ayant au moins l'un d'une tolérance altérée au glucose et d'un diabète de type 2 lorsque le rapport phénotypique de SAA est modulé par une quantité statistiquement significative par rapport à un rapport phénotypique de SAA présent chez un ou plusieurs sujets ayant une tolérance au glucose normale comprend l'étape de diagnostic du sujet comme ayant une tolérance altérée au glucose lorsque le rapport phénotypique du SAA dans l'échantillon biologique du sujet est d'au moins 1,3760.

6. Procédé selon la revendication 1, dans lequel l'étape de diagnostic dudit sujet comme ayant au moins l'un d'une tolérance altérée au glucose et d'un diabète de type 2 lorsque le rapport phénotypique de SAA est modulé par une quantité statistiquement significative par rapport à un rapport phénotypique de SAA présent chez un ou plusieurs sujets ayant une tolérance au glucose normale comprend l'étape de diagnostic du sujet comme ayant un diabète de type 2 lorsque le rapport phénotypique du SAA dans l'échantillon biologique du sujet est d'au moins 1,4780.

7. Procédé selon la revendication 1, dans lequel l'étape de diagnostic dudit sujet comme ayant au moins l'un d'une tolérance altérée au glucose et d'un diabète de type 2 comprend l'étape de diagnostic du sujet comme ayant au moins l'un d'une tolérance altérée au glucose et d'un diabète de type 2 lorsque le rapport phénotypique de SAA dans l'échantillon biologique du sujet est augmenté d'une quantité statiquement significative par rapport à un niveau présent dans l'échantillon biologique d'un ou de plusieurs sujets ayant une tolérance au glucose normale.

8. Procédé selon la revendication 7, dans lequel l'étape de diagnostic dudit sujet comme ayant au moins l'un d'une tolérance altérée au glucose et d'un diabète de type 2 lorsque le rapport phénotypique de SAA est modulé par une quantité statistiquement significative par rapport à un rapport phénotypique de SAA présent chez un ou plusieurs sujets ayant une tolérance au glucose normale comprend l'étape de diagnostic du sujet comme ayant une tolérance altérée au glucose lorsque le rapport phénotypique du SAA dans l'échantillon biologique du sujet est d'au moins 1,3760.

9. Procédé selon la revendication 7, dans lequel l'étape de diagnostic dudit sujet comme ayant au moins l'un d'une tolérance altérée au glucose et d'un diabète de type 2 lorsque le rapport phénotypique de SAA est modulé par une quantité statistiquement significative par rapport à un rapport phénotypique de SAA présent chez un ou plusieurs sujets ayant une tolérance au glucose normale comprend l'étape de diagnostic du sujet comme ayant un diabète de type 2 lorsque le rapport phénotypique du SAA dans l'échantillon biologique du sujet est d'au moins 1,4780.

10. Procédé selon la revendication 8, dans lequel l'étape de détermination d'un rapport phénotypique de SAA dans l'échantillon biologique du sujet comprend l'étape de réalisation d'un seul dosage.

11. Procédé selon la revendication 10, dans lequel l'étape de réalisation d'un seul dosage comprend l'étape de réalisation d'une seule spectrométrie de masse.

12. Procédé selon la revendication 9, dans lequel l'étape de détermination d'un rapport phénotypique de SAA dans l'échantillon biologique du sujet comprend l'étape de réalisation d'un seul dosage.

13. Procédé selon la revendication 12, dans lequel l'étape de réalisation d'un seul dosage comprend l'étape de réalisation d'une seule spectrométrie de masse.
